(19) Europäisches Patentamt
European Patent Office
Office européen des brevets

(11) Publication number: 0 162 494
A1

# (12) EUROPEAN PATENT APPLICATION

(21) Application number: 85200549.5

(22) Date of filing: 10.04.85

(51) Int. Cl.[4]: C 07 D 275/06
A 01 N 43/80

(30) Priority: 13.04.84 NL 8401184

(43) Date of publication of application:
27.11.85 Bulletin 85/48

(84) Designated Contracting States:
AT BE DE FR GB IT NL SE

(71) Applicant: AKZO N.V.
Velperweg 76
NL-6824 BM Arnhem(NL)

(72) Inventor: Bronsdijk, Johannes Marinus
Horstlindelaan 100
NL-7522 JK Enschede(NL)

(72) Inventor: Praetorius, Heinz August
Verdiweg 409
NL-3816 KM Amersfoort(NL)

(74) Representative: Sieders, René et al,
P.O. Box 314
NL-6800 AH Arnhem(NL)

## (54) Process for the preparation of a 3-hydroxy-2,3-dihydrobenzisothiazole 1,1-dioxide and the use of this compound as a biocide and for preparing an ortho-sulphobenzimide.

(57) The invention relates to a one-step process for the preparation of a 3-hydroxy-2,3-dihydrobenzisothiazole-1,1-dioxide in a high yield by oxidation of an ortho-toluene sulphonamide in the liquid phase using molecular oxygen and/or ozone in the presence of one or more catalysts.

The oxidation is terminated before 50% of the starting material has been convented into resultant oxidation products from the 3-hydroxy-2,3-dihydrobenzisothiazole-1,1-dioxide and into other oxidation products. It is preferred that the reaction should be carried out in acetic acid or free of solvent using cobalt and/or manganese salts as a catalyst.

The benzisothiazole-1,1-dioxides can be oxidized to an ortho-sulphobenzimide. In this way saccharin can be prepared. The bezisothiazole-1,1-dioxides also display biocidal activity.

EP 0 162 494 A1

./...

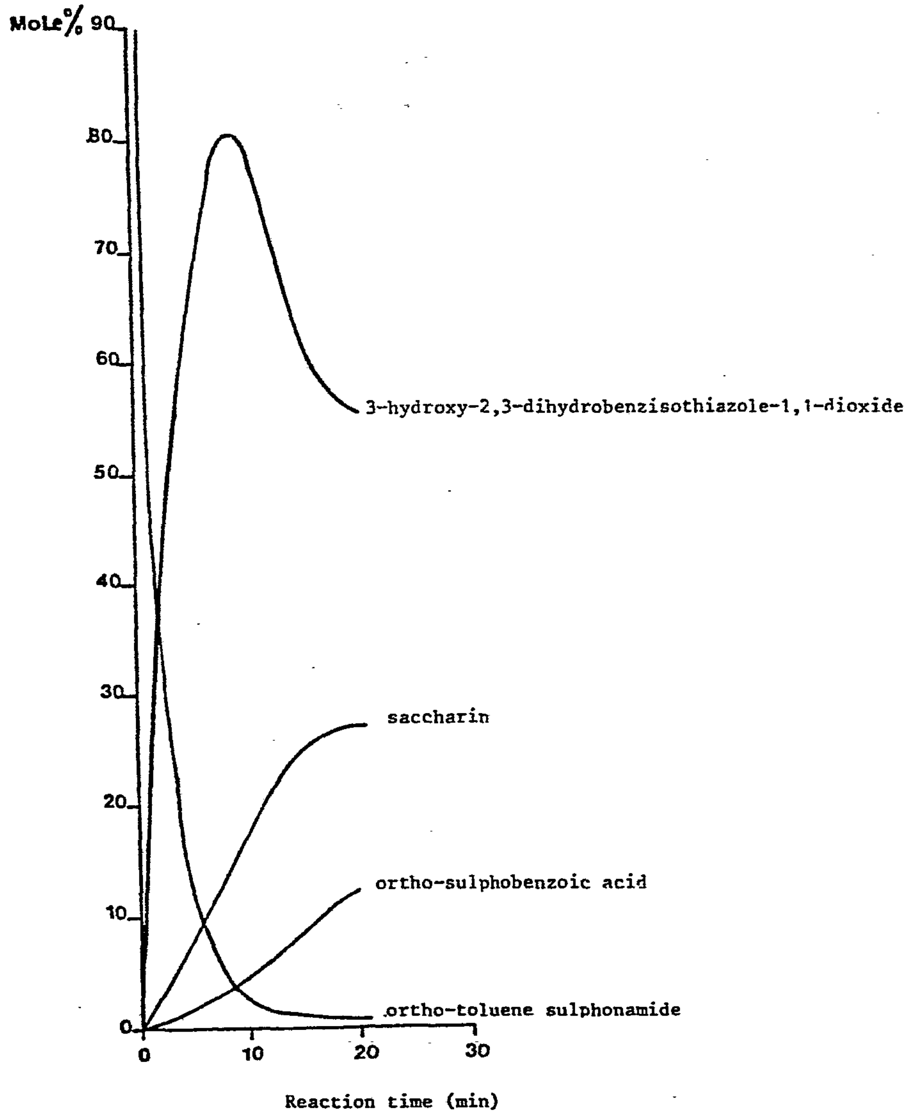
MoLe% 90
80
70
60
50
40
30
20
10
0
3-hydroxy-2,3-dihydrobenzisothiazole-1,1-dioxide
saccharin
ortho-sulphobenzoic acid
ortho-toluene sulphonamide
0
10
20
30
Reaction time (min)

fig 1

Process for the preparation of a 3-hydroxy-2,3-dihydrobenziso-thiazole-1,1-dioxide and the use of this compound as a biocide and for preparing an ortho-sulphobenzimide

The invention relates to a process for the preparation of a 3-hydroxy-2,3-dihydrobenzisothiazole-1,1-dioxide by oxidation in the liquid phase of an ortho-toluene sulphonamide. A process of the above type is known from Helv. Chem. Acta, 12, 669 (1929). It describes a process in which by a multi-step synthesis a derivative of 3-hydroxy-2,3-dihydrobenzisothiazole-1,1-dioxide can be obtained in very low yield from an ortho-toluene sulphonamide.
A method has now been found by which in a one-step process a high yield conversion can be realized.

The invention consists in that in a process of the type indicated above the oxidation is carried out using molecular oxygen and/or ozone in the presence of one or more catalysts, and the formation of byproducts is limited by terminating the oxidation before 50% of the starting material has been converted into resultant oxidation products from the 3-hydroxy-2,3-dihydrobenzisothiazole-1,1-dioxide and into other oxidation products.

The reaction may be represented as follows:

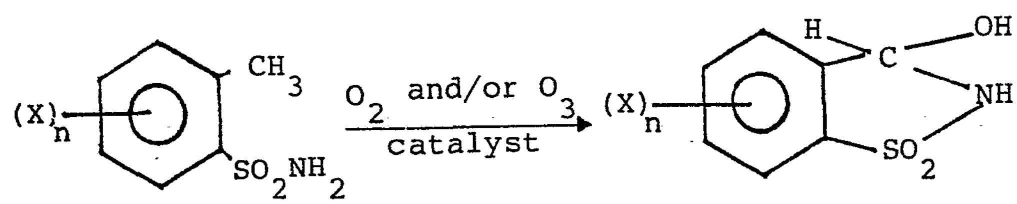


wherein the group X is an aromatic ring substituent; such as an alkyl group, an acyl group, a carboxyl group, a nitro group or a halogen atom. When X = alkyl, the reaction will generally differ from the above representation in that the alkyl groups will be (partially) oxidized. The value of n is 0, 1 or 2.

As oxidation agent molecular oxygen and/or ozone may be used, optionally inert gas may be present. The reaction is carried out in the liquid phase and in the presence or not of a solvent. Examples of suitable solvents include aliphatic carboxylic acids which may be esterified and/or halogenated, such as acetic acid, propionic acid, ethyl acetate, 2-bromoproprionic acid and methyl chloroacetate.

It is preferred that the reaction should be carried out in acetic acid or free of solvent. The amount of the ortho-toluene sulphonamide which is dissolved in acetic acid is generally in the range of 3 to 20 per cent by weight.

To increase the rate of the reaction the process is carried out in the presence of at least one catalyst which is suitable for use in the oxidation of alkylaromatic hydrocarbons. Examples of suitable catalysts include cobalt salts, manganese salts, iron salts and nickel salts of organic or inorganic acids.

A preferred catalyst is cobalt-II-bromide or cobalt-II-acetate to which a small amount of hydrogen bromide is added.

The presence of a manganese-salt in combination with a cobalt-salt has a favourable effect on the rate and the selectivity of the oxidation reaction.

The duration of the reaction is very much dependent on the choice of the temperature, pressure and oxygen concentration.

The reaction will generally be carried out at a temperature in the range of 100° to 200° C and a pressure of 1 to 30 bar. The preparation may be carried out batch-wise or in a continuous process.

Essential to the invention is that the reaction is stopped before 50% of the (substituted) ortho-toluene sulphonamide has been converted into resultant oxidation products from the (substituted) 2,3-dihydrobenzisothiazole-1,1-dioxide and into other oxidation products. The oxidation is preferably stopped when 90 to 99% of the ortho-toluene sulphonamide has been converted.

In this way the formation of byproducts is curbed, which is illustrated in Fig. 1.

This figure shows that the conversion of sulphonamide and the formation of benzisothiazole initially proceed at a high speed. The formation of benzisothiazole, however, will reach a maximum value and upon continuation of the oxidation the amount of benzisothiazole will rapidly decrease. Important byproducts that are formed then are saccharin and ortho-sulphobenzoic acid.
It should be added that NL 7 103 967 describes a process for the preparation of ortho-sulphobenzimides, such as saccharin, by oxidation of ortho-toluene sulphonamides with oxygen in the presence of catalysts.

The present benzisothiazole-1,1-dioxides are also very suitable starting materials for the preparation of ortho-sulphobenzimides by oxidation. Examples of suitable oxidation agents include hydrogen peroxide, potassium permanganate and chromium trioxide. It is preferred that use should be made of hydrogen peroxide. Oxidation may be carried out in an aqueous medium at atmospheric pressure and a temperature in the range of 25° to 100° C, preferably 60° to 95° C. The ortho-sulphobenzimides can be converted into a salt, e.g. a sodium salt.
Preferably saccharin or the sodium salt therefrom is prepared.

The invention also relates to another use of the present benzothiazole-1,1-dioxides.
It has been found that these compounds are particularly suitable for use as a biocide or for the preparation of a composition having biocidal activity. It appears that 3-hydroxy-2,3-dihydrobenzothiazole-1,1-dioxide displays better bacteriostatic and fungistatic activity than 3-methoxy-1,2-benzisothiazole-1,1-dioxide disclosed in US 3 629 428, as will be demonstrated in Example 3.
The minimum growth inhibiting concentration (MIC value) of 3-hydroxy-2,3-dihydrobenzisothiazole-1,1-dioxide for the different types of microorganisms was determined in conformity with the dilution test method of the "Deutsche Gesellschaft für Hygiene und Mikrobiologie" (DGHM) and are:

| | | |
|---|---|---|
| gram negative bacteria | : | 600 - 2400 ppm |
| gram positive bacteria | : | 600 - 1200 ppm |
| yeasts | : | 300 - 1200 ppm |
| fungi | : | 37,5 - 600 ppm. |

The compound displays a low oral toxicity, a remarkable bacteriostatic activity towards sulfphate reducing bacteria and in combination with some quaternary ammonium compounds such as dimethylbenzyl coco-ammonium chloride or didecyldimethyl ammonium chloride it has a synergistic effect relative to certain bacteria. The present biocides are particularly suitable to be used under anaerobic conditions.

If required, the 3-hydroxy-2,3-dihydrobenzisothiazole-1,1-dioxide can be used in the form of a salt or a derivative in which the hydroxy group is replaced by for instance a methoxy group (see example 2). Such compounds easily hydrolyse back to the 3-hydroxy-2,3-dihydrobenzisothiazole.

The 2,3-dihydrobenzisothiazole-1,1-dioxide may be used undiluted or in combination with a liquid or solid diluent.

It is preferred that the diluent should be chemically inert relative to the biocide.

Examples of suitable solid diluents (or carriers) are clay, kaolin, talc, silica, vermiculite, etc.

Liquid diluents are liquids in which the biocide can be dissolved, emulsified of dispersed, in the presence optionally of a surface active agent.

As examples of suitable diluents may be mentioned water, kerosine, dioxane, acetone, animal and vegetable fats.

The effectiveness of the biocide may be promoted by adding one or more agents, such as a spreading agent, a wetting agent, a sticking agent and an emulsifying agent. Depending on its field of application the biocide may be used in combination with other biologically active compounds such as bactericides, fungicides, herbicides, insecticides and antibiotics.

The concentration of the 2,3-dihydrobenzisothiazole-1,1-dioxide in the total composition may range within wide limits. In liquid compositions generally a concentration of 50 to 2000 ppm is adhered to and in solid compositions a concentration of 0,5 to 20 percent by weight.
The present biocide may be used to control undesirable bacteria, yeasts and fungi both under aerobic and anaerobic conditions. Fields of application include agriculture, fruit growing and bulb farming. The present biocides are also very suitable to control sulphate reducing bacteria in water systems.
The invention will be further described in the following examples.

Example 1

3-Hydroxy-2,3-dihydrobenzisothiazole-1,1-dioxide was prepared as follows. To acetic acid (2800 g) there were added at 80° C: ortho-toluene sulphonamide (390 g), cobalt-II-acetate (21 g), manganese-II-acetate (2,5 g) and hydrogen bromide (32 g of a 47%-solution in water). The mixture was charged into an autoclave and pressure was applied (nitrogen 9 bar and on top of which afterwards oxygen 6 bar). Subsequently, the reaction mixture was heated, with stirring, to a temperature of 120° C, which was attended with rapid take up of oxygen and the temperature rising to 190° C. During the reaction oxygen was supplied at a rate such that in the autoclave a pressure was maintained which consisted of an initial pressure corrected for the temperature increased by the vapour pressure of acetic acid. One and a half minutes after a reaction temperature of 190° C had been attained, the reaction was terminated by rapid cooling. The total reaction time was 6 minutes. It was found that at a conversion of 96% of the ortho-toluene sulphonamide 74% had been converted into 3-hydroxy-2,3-dihydrobenzisothiazole-1,1-dioxide.

Example 2

Optionally, the 3-hydroxy-2,3-dihydrobenzisothiazole-1,1-dioxide may be purified as follows.

The product mixture of Example 1 was concentrated by evaporation under reduced pressure (15-20 mm Hg, 50°-60° C). The resulting concentrate contained about 20% of acetic acid and 55% of 3-hydroxy-2,3-dihydrobenzisothiazole-1,1-dioxide. The concentrate was refluxed for 2 hours in methanol (1700 g). Upon cooling the solution, 3-methoxy-2,3-dihydrobenzisothiazole-1,1-dioxide crystallized. The mother liquor could be used to convert a next batch of the concentrate. In this way 3-methoxy-2,3-dihydrobenzisothiazole-1,1-dioxide could be obtained in a yield of over 95%. This compound (150 g) was subsequently refluxed for 2 hours in demineralized water (4 l), with the compound going into solution. Upon cooling the solution 3-hydroxy-2,3-dihydrobenzisothiazole-1,1-dioxide crystallized. The crystals were washed with water and dried at 25° C. The yield of 3-hydroxy-2,3-benzisothiazole-1,1-dioxide was 54%, the purity 98-99%. The mother liquor could be used for a next hydrolysis, as a result of which the yield increased from 54% to almost 100%, without any detrimental effect on the purity of the product.

<u>Example 3</u>

Ortho-toluenesulphonamide was dissolved in acetic acid and after addition of cobalt-II-acetate, manganese-II-acetate and hydrogen bromide it was oxidized with oxygen. The product mixture (500 g), which contained 248 g of 3-hydroxy-2,3-dihydrobenzisothiazole-1,1-dioxide, was evaporated off at 60° C and a pressure of 2 to 3 mm Hg. Previously, a little water had been added to avoid side reactions. Subsequently, 304 g of hydrogen peroxide (30%) were added to the mixture over a period of 2 hours at 80° C for 1 more hour, it was cooled and the crystalline saccharin was filtered and dried (saccharin yield 250 g, purity 95%, efficiency 97%).

<u>Example 4</u>

The bacteriostatic and fungistatic action of 3-hydroxy-2,3-dihydrobenzisothiazole-1,1-dioxide and the 3-methoxy-1,2-benzisothiazole-1,1-dioxide described in US 3 629 428 was determined by a modified dilution method of the "Deutsche Gesellschaft für

Hygiene und Mikrobiologie" (DGHM) and is described in "Richtlinien für die Prüfung und Bewerkung chemischer Desinfektionsverfahren", pp. 4-5, Gustav Fischer, Stuttgart, 1981.
The MIC values determined in this way are given in the table below, which shows that 3-hydroxy-2,3-dihydrobenzisothiazole-1,1-dioxide has a higher bacteriostatic and a far higher fungistatic activity than 3-methoxy-1,2-benzisothiazole-1,1-dioxide.

| Micro-organism | 3-hydroxy 2,3-dihydro benzisothiazole 1,1-dioxide | 3-methoxy 1,2-benzisothiazole 1,1-dioxide |
|---|---|---|
| | MIC-values in ppm (A.C.)* | |
| Gram negative bacteria | | |
| Pseudomonas aeruginosa | 1200 | > 1200 |
| Escherichia coli | 600-1200 | 1200 |
| Proteus mirabilis | 600-1200 | 600-1200 |
| Gram positive bacteria | | |
| Staphylococcus aureus | 600-1200 | > 1200 |
| Streptococcus faecalis | 600-1200 | > 1200 |
| Yeasts | | |
| Candida albicans | 600-1200 | > 1200 |
| Saccharomyces cerevisiae | 300- 600 | > 1200 |
| Fungi | | |
| Chaetomium globosum | 37,5- 75 | > 1200 |
| Paecillomyces varioti | 18,8- 37,5 | 600-1200 |
| Trichoderma viride | 75 -150 | > 1200 |
| Aspergillus niger | 75 -150 | > 1200 |
| Pellicularia sasaki | 150 | 600-1200 |
| Pyricularia oryzae | 150 -200 | > 1200 |

* A.C. = active compound

Example 5

The acute oral toxicity of 3-hydroxy-2,3-dihydro-1,2-benzisothiazole-1,1-dioxide was determined on BOR-WISW (SPF TNO) Wistar rats. Groups of 5 ♂ and 5 ♀ rats were administered 0,5, 1,0 and 2,5 g/kg of the compound. The calculation of the oral $LD_{50}$ was performed in accordance with D.Y. Finney, Probit Analysis, 3rd Ed., Cambridge 1971.

| $LD_{50}$ (g/kg) of | after 24 hours | after 48 hours | after 14 days |
|---|---|---|---|
| ♂ | 1,38 | 1,22 | 1,22 |
| ♀ | 0,86 | 0,86 | 0,86 |
| ♂ + ♀ | 1,07 | 1,02 | 1,02 |

Claims

1. A process for the preparation of a 3-hydroxy-2,3-dihydrobenzisothiazole-1,1-dioxide by oxidation in the liquid phase of an ortho-toluene sulphonamide, characterized in that the oxidation is carried out using molecular oxygen and/or ozone in the presence of one or more catalysts, and the formation of byproducts is inhibited by terminating the oxidation before 50% of the starting material has been converted into resultant oxidation products from the 3-hydroxy-2,3-dihydrobenzisothiazole-1,1-dioxide and into other oxidation products.

2. A process according to claim 1, characterized in that the oxidation is terminated at a conversion of the sulphonamide in the range of 90 to 99%.

3. A process according to claim 1 or 2, characterized in that the catalyst system comprises cobalt salts and/or manganese salts.

4. A process according to claim 1, 2 or 3, characterized in that the liquid phase substantially consists of acetic acid.

5. A process according to any one of the preceding claims, characterized in that the ortho-toluene sulphonamide is unsubstituted.

6. The use of a benzisothiazole-1,1-dioxide obtained according to any one of the preceding claims for the preparation of an ortho-sulphobenzimide or a salt thereof.

7. The use according to claim 6, characterized in that a benzisothiazole-1,1-dioxide is converted into an ortho-sulphobenzimide by means of hydrogen peroxide.

8. The use of a benzisothiazole-1,1-dioxide according to any of the claims 1 to 5 as a biocide or for the preparation of a composition which has a biocidal activity.

ACH 1928
0162494

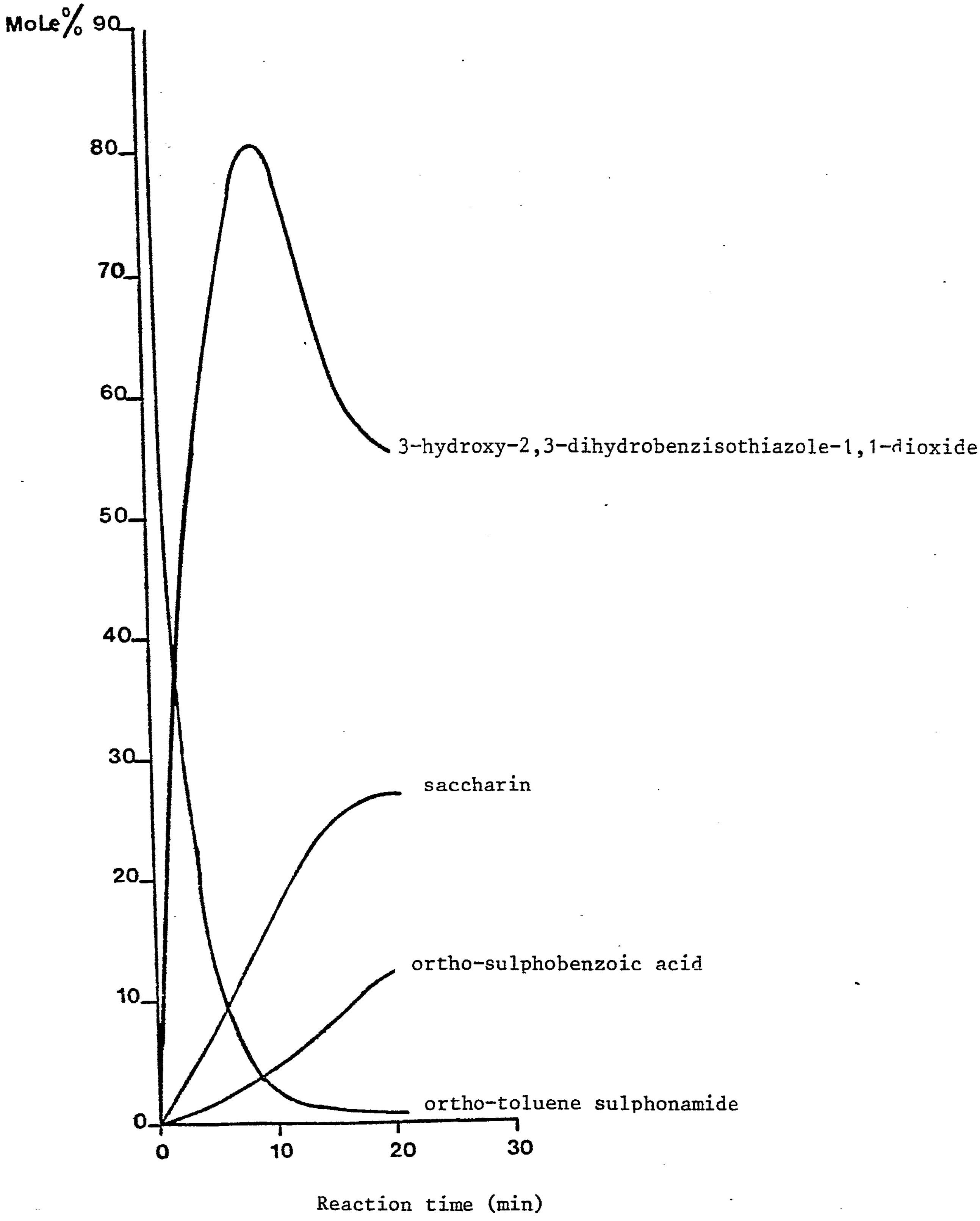


fig 1

European Patent Office

# EUROPEAN SEARCH REPORT

0162494

Application number

EP 85 20 0549

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X,D | NL-A-7 103 967 (RHONE-POULENC) * Claims * | 6,7 | C 07 D 275/06<br>A 01 N 43/80 |
| A | | 1-5 | |
| | --- | | |
| A | US-A-3 629 428 (S. SEKI et al.) | 8 | |
| | ----- | | |
| | | | TECHNICAL FIELDS SEARCHED (Int. Cl.4) |
| | | | C 07 D<br>A 01 N |

The present search report has been drawn up for all claims

| Place of search | Date of completion of the search | Examiner |
|---|---|---|
| THE HAGUE | 23-07-1985 | DECORTE D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82